(19) Europäisches Patentamt
European Patent Office
Office européen des brevets

(11) Publication number: **0 022 515**
**B1**

(12) **EUROPEAN PATENT SPECIFICATION**

(45) Date of publication of patent specification: **03.08.83**

(51) Int. Cl.³: **C 07 H 15/24, A 61 K 31/70**

(21) Application number: **80103743.3**

(22) Date of filing: **01.07.80**

# (54) Anthracycline glycosides, process for their preparation and therapeutical composition containing them.

(30) Priority: **04.07.79 GB 7923225**

(43) Date of publication of application:
**21.01.81 Bulletin 81/3**

(45) Publication of the grant of the patent:
**03.08.83 Bulletin 83/31**

(84) Designated Contracting States:
**AT BE CH DE FR GB LI NL SE**

(56) References cited:
**BE - A - 864 336**
**FR - A - 2 389 640**
**GB - A - 2 016 005**

**JOURNAL OF MEDICINAL CHEMISTRY, vol. 22, no. 2, February 1979, pages 121—123, American Chemical Society**
**G. CASSINELLI: "Synthesis and Antitumor Activity of 4'-O-Methyladriamycin and Their 4'-Epi Analogues"**

(73) Proprietor: **FARMITALIA CARLO ERBA S.p.A.**
**Via Carlo Imbonati n.24**
**I-20159 Milan (IT)**

(72) Inventor: **Cassinelli, Giuseppe**
**Via G. Matteotti, 13**
**Voghera (PV) (IT)**
Inventor: **Forenza, Salvatore**
**Via R. Gessi, 48**
**Milan (IT)**
Inventor: **Ripamonti, Maria Clara**
**Via Edison, 1**
**Monza (MI) (IT)**
Inventor: **Ruggieri, Daniela**
**Via Giovanni da Procida, 21**
**Milan (IT)**

(74) Representative: **Hansen, Bernd, Dr.rer.nat. et al,**
**Hoffmann . Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Anthracycline glycosides, process for their preparation and therapeutical composition containing them

The invention relates to anthracycline glycosides, a process for their preparation and a therapeutical composition containing them.

The invention provides anthracycline glocysides of the general formula (I)

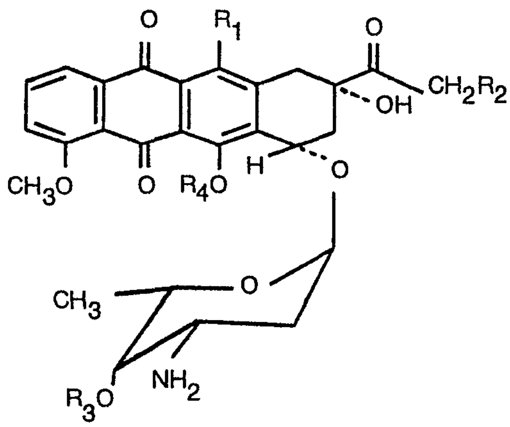

(I)

wherein $R_1$ represents a hydrogen atom or a methoxy group, $R_2$ represents a hydrogen atom or a hydroxy group, and each of $R_3$ and $R_4$ independently represents a hydrogen atom or a methyl group, with the proviso that $R_3$ and $R_4$ do not simultaneously represent hydrogen atoms, and their pharmaceutically acceptable acid addition salts.

Clearly when $R_2$ represents a hydrogen atom the compounds are daunorubicin analogues while when $R_2$ represents a hydroxy gorup they are doxorubicin analogues. Particular reference will be made to 4'-O-methyl-11-deoxy-daunorubicin (II), 4'-O-methyl-11-deoxy-doxorubicin (III), 4',6-di-O-methyl-11-deoxy-daunorubicin (IV), 4',6-di-O-methyl-11-deoxy-doxorubicin (V), 11-O-methyl-daunorubicin (VI), 11-O-methyl-doxorubicin (VII), 4',11-di-O-methyl-daunorubicin (VIII) and 4',11-di-O-methyl-doxorubicin (IX), each of which has the above formula I with substituents as follows:

| | | | |
|---|---|---|---|
| II) | $R_1=R_2=R_4=H$; $R_3=CH_3$ | VI) | $R_1=OCH_3$; $R_2=R_3=R_4=H$ |
| III) | $R_1=R_4=H$; $R_2=OH$; $R_3=CH_3$ | VII) | $R_1=OCH_3$; $R_2=OH$; $R_3=R_4=H$ |
| IV) | $R_1=R_2=H$; $R_3=R_4=CH_3$ | VIII) | $R_1=OCH_3$; $R_2=R_4=H$; $R_3=CH_3$ |
| V) | $R_1=H$; $R_2=OH$; $R_3=R_4=CH_3$ | IX) | $R_1=OCH_3$; $R_2=OH$; $R_3=CH_3$; $R_4=H$ |

Compounds (VI) and (VII) are disclosed in applicant's Belgian Patent Specification No 864336.

The invention further provides a process for the preparation of the compounds of the present invention, the process comprising N-trifluoroacetylating 11-deoxy-daunorubicin or daunorubicin, methylating the resultant N-trifluoroacetyl derivative respectively, removing the N-trifluoroacetyl group from the resultant methylated product, and optionally 14-brominating and subsequent hydrolysing to produce doxorubicin compounds, wherein $R_2$=OH and, if desirable, reacting the respective products with acid to obtain the pharmaceutically acceptable acid addition salt.

One of the starting compounds, i.e. 11-deoxy-daunorubicin, a neutral anthracycline, is described in applicant's Belgian Patent No. 874032.

The process of N-trifluoroacetylation, methylation and de-N-trifluoroacetylation caried out on daunorubicin leads to 11-O-methyl-daunorubicin (VI) and 4',11-di-O-methyl-daunorubicin (VIII); while the similar process with 11-deoxy-daunorubicin gives rise to 4'-O-methyl-11-deoxy-N-trifluoroacetyl-daunorubicin and 4',6-di-O-methyl-11-deoxy-N-trifluoroacetyl-daunorubicin which then are de-N-trifluoroacetylated respectively.

These processes are partly illustrated with reference to the formulae and reaction schemes below wherein the specific methylating agent should not be construed in a restrictive way.

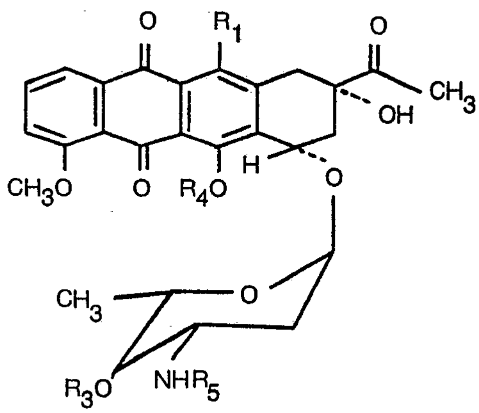

X $R_1=R_3=R_4=H$, $R_5=COCF_3$
11-deoxy-N-trifluoroacetyl-daunorubicin

XI $R_1=OH$, $R_3=R_4=H$, $R_5=COCF_3$
N-trifluoroacetyl-daunorubicin

XII $R_1=R_4=H$, $R_3=CH_3$, $R_5=COCF_3$
4′-O-methyl-11-deoxy-N-trifluoroacetyl-daunorubicin

XIII $R_1=H$, $R_3=R_4=CH_3$, $R_5=COCF_3$
4′,6-di-O-methyl-11-deoxy-N-trifluoroacetyl-daunorubicin

XIV $R_1=OCH_3$, $R_3=R_4=H$, $R_5=COCF_3$
11-O-methyl-N-trifluoroacetyl-daunorubicin

XV $R_1=OCH_3$, $R_3=CH_3$, $R_4=H$, $R_5=COCF_3$
4′,11-di-O-methyl-N-trifluoroacetyl-daunorubicin

XVI $R_1=R_3=R_4=R_5=H$
11-deoxy-daunorubicin

XVII $R_1=OH$, $R_3=R_4=R_5=H$
daunorubicin

XVI —N-trifluoroacetylation→ X —$CH_3I$ / $Ag_2O$→ XII + XIII
XII —mild alkaline hydrolysis→ II
XIII —mild alkaline hydrolysis→ IV

XVII —N-trifluoroacetylation→ XI —$CH_3I$ / $Ag_2O$→ XIV + XV
XIV —mild alkaline hydrolysis→ VI
XV —mild alkaline hydrolysis→ VIII

N-trifluoroacetyl-daunorubicin (XI) is known from applicant's United States Patent Specification No. 3808124 and the preparation of (VI) and (VIII) may commence from this compound, omitting the first step of N-trifluoroacetylating daunorubicin (XVII).

Subsequent 14-bromination and hydrolysis of the daunorubicin analogues II, IV, VI and VIII, preferably according to the method described in applicant's United States Patent Specification No. 3,803,124, affords the corresponding doxorubicin analogues III, V, VII and IX, and this conversion is also within the scope of the invention.

A preferred embodiment of the present process comprises reacting the known 11-deoxy-daunorubicin or daunorubicin with trifluoroacetic anhydride in anhydrous chloroform to obtain their respective

N-trifluoroacetyl derivatives which are successively and separately treated with methyl iodide in the presence of silver oxide, separating by chromatography on a silica gel column and using a chloroform-methanol gradient solution the so formed 4'-O-methyl-11-deoxy-N-trifluoroacetyl-daunorubicin from the contemporaneously formed 4',6-di-O-methyl-11-deoxy-N-trifluoroacetyl-daunorubicin or the 11-O-methyl-daunorubicin from the 4'-11-di-O-methyl-daunorubicin, removing separately from each compound the protecting N-trifluoroacetyl groups by mild alkaline hydrolysis to obtain the free glycosidic bases ($R_2$=H) which, by treatment with methanolic hydrogen chloride are isolated as their hydrochlorides and eventually reacted with bromine in chloroform to obtain their respective 14-bromo-derivatives, which, by subsequent hydrolysis at room temperature with a solution of sodium formate give rise to the doxorubicin derivatives of the general formula I ($R_2$=OH) also isolated as their hydrochlorides.

The compounds according to the present invention exhibit marked pharmacological activity which in form of therapeutical compositions are useful namely as antitumor drugs.

BIOLOGICAL ACTIVITY DATA

Antitumor activity

The new compounds have been tested on HeLa cells cloning efficiency *in vitro* and on P—388 ascitic leukemia in mice, in comparison with daunorubicin and doxorubicin.

Data reported in Table 1 show that 11-deoxy-4'-O-methyl derivatives of daunorubicin (*II*) and of doxorubicin (III) are less cytotoxic than their parent compounds. Accordingly, they were also less toxic *in vivo* and at the maximal tolerated dose showed an antitumor activity comparable to that of the parent compounds (Table 2). Of particular interest is compound III which showed a large range of active and non toxic doses.

Compounds *VI* and *VIII* were markedly less cytotoxic than the parent compounds (Table 1) and when tested *in vivo* even at the dose of 200 mg/kg, they were not toxic and displayed an antitumor activity comparable to that of daunorubicin in the same experiment (Table 2).

TABLE 1

Effect on HeLa cells cloning efficiency in vitro [a)]

| Compound | $ID_{50}$ (ng/ml) |
|---|---|
| Daunorubicin • HCl | 7.2 |
| Doxorubicin • HCl (adriamycin) | 9.5 |
| 4'-O-methyl-11-deoxy-daunorubicin • HCl (II) | 66 |
| 4'-O-methyl-11-deoxy-doxorubicin • HCl (III) | 22 |
| 4'-6-di-O-methyl-11-deoxy-daunorubicin • HCl (IV) | 1000 |
| 11-O-methyl-daunorubicin • HCl (VI) | 400 |
| 4'-11-di-O-methyl-daunorubicin • HCl (VIII) | 500 |

a) HeLa cells were exposed to the drugs for 24 hours, then plated. Colonies number was evaluated 5 days later.

TABLE 2

Antitumor activity on ascitic P 388 leukemia in mice

| Compound | Dose (a) mg/kg | T/C (b) % | Toxic deaths (c) |
|---|---|---|---|
| Daunorubicin • HCl (d) | 2.9<br>4.4<br>6.6 | 175,122<br>180,122<br>165,122 | 0/10, 0/8<br>3/10, 0/6<br>8/10, 0/6 |
| 4'-O-methyl-11-deoxy-daunorubicin • HCl (II) | 25<br>50 | 180<br>225 | 0/9<br>1/9 |
| 4'-11-di-O-methyl-daunorubicin • HCl (VIII) | 100<br>200 | 111<br>122 | 0/6<br>0/6 |
| 11-O-methyl-daunorubicin • HCl (VI) | 100<br>200 | 111<br>122 | 0/4<br>0/3 |
| Doxorubicin • HCl (Adriamycin) | 4.4<br>6.6<br>10.0 | 219<br>223<br>180 | 0/8<br>0/8<br>4/8 |
| 4'-O-methyl-11-deoxy-doxorubicin • HCl (III) | 6.6<br>10<br>15<br>22.5 | 195<br>209<br>238<br>214 | 0/8<br>0/8<br>0/7<br>0/10 |

(a) Mice were treated i.p. on day 1 after tumor cells inoculation.

(b) Median survival time of treated mice/median survival time of control mice × 100.

(c) Evaluated on the base of the macroscopic autoptic findings.

(d) Data of two experiments.

The preparation of the compounds of the present invention is illustrated by the following examples:

Example 1

Preparation of 4'-O-methyl-11-deoxydaunorubicin (II) (IMI—108)

A solution of 0.600 (1.09 mmoles) of 11-deoxy-daunorubicin hydrochloride in anhydrous chloroform (60 ml) was treated with trifluoroacetic anhydride (2.0 ml) at 0°C, under stirring. After 1 hour the resulting solution was evaporated to dryness under reduced pressure and the residue was suspended in water (50 ml), adjusted to pH 8 with saturated aqueous sodium bicarbonate and extracted with chloroform (2 x 50 ml).

The organic phase was separated, dried over anhydrous sodium sulphate and reduced to small volume (≃5 ml). 100 ml of methanol was added. After 2 hours the resulting solution was evaporated to dryness, the residue dissolved in the minimum of chloroform needed and addition of sufficient petroleum ether precipitated 0.630 g (1.05 mmoles) of 11-deoxy-N-trifluoroacetyl-daunorubicin (X) as a yellow crystalline powder: m.p. 147—149°C; $[\alpha]_D = +140°$ (c = 0.1 in MeOH); m/e 607 ($M^+$). The p.m.r. spectrum ($CDCl_3$) shows absorptions at 129 (d, $CH_3$—C—5'), 2.37 (s, $CH_3$—CO), 4.03 (s, C—4—$OCH_3$), 5.22 (broad signal, C—7—H), 5.47 (broad signal, C—1'—H) and 13.80δ (s, C—6—OH).

Compound X (0.650 g; 1.07 mmoles was treated with methyl iodide (50 ml), in the presence of silver oxide (1.3 g) at 40°C for 2 hours, under stirring. The reaction mixture was filtered, the solid washed with chloroform (3 x 100 ml) and the combined filtrates evaporated to dryness under reduced pressure. The crude reaction mixture was purified on a silica gel column, eluting with a chloroform methanol gradient solution, to afford 4'-O-methyl-11-deoxy-N-trifluoroacetyl-daunorubicin (XII) (0.250 g; 0.41 mmoles) and 4',6-di-O-methyl-11-deoxy-N-trifluoroacetyl-daunorubicin (XIII) (0.200 g; 0.31 mmoles).

Compound XII (eluted with chloroform) has m.p. 128—131°C; $[\alpha]_D = +140°$ (c = 0.1 in $CH_3OH$); m/e 621 ($M^+$). The p.m.r. spectrum ($CDCl_3$) shows absorptions at 1.37 (d, $CH_3$—C—5'), 2.40 (s, $CH_3$—CO), 3.57 (s, C—4'—$OCH_3$), 4.08 (s, C—4—$OCH_3$), 5.30 (broad signal, C—7—H), 5.53 (broad signal, C—1'—H) and 13.70δ (s, C—6—OH).

Compound XIII (eluted with chloroform-methanol 99:1) has m.p. 124—127°C; $[\alpha]_D = +25°$ (c = 0.1 in $CH_3OH$); m/e 635 ($M^+$). The p.m.r. spectrum ($CDCl_3$) shows absorptions at 1.37 (d, $CH_3$—C—5'), 2.38 (s, $CH_3CO$), 3.55 (s, C—4'—$OCH_3$), 4.02 (s, two aromatic $OCH_3$), 5.33 (broad signal, C—7—H) and 5.43δ (broad signal, C—1'—H).

A solution of compound XII (0.230 g; 0.37 mmoles) in 0.25 M aqueous sodium hydroxide (25 ml) was kept for 30 minutes at room temperature, under stirring. The resulting solution was diluted with water (30 ml), adjusted to pH 3 with 0.25 M hydrochloric acid, and extracted with chloroform to eliminate inpurities. The aqueous phase, adjusted to pH 8.2 with saturated aqueous sodium bicarbonate was extracted with chloroform (3 x 100 ml). The combined organic extracts were dried over anhydrous sodium sulphate, concentrated to small volume and acidified to pH 4.5 with 0.5 M methanolic hydrogen chloride. Addition of sufficient petroleum ether precipitated 4'-O-methyl-11-deoxy-daunorubicin (II) as its hydrochloride (0.200 g; 0.36 mmoles): m.p. 194—197°C (with decomposition); mass spectrum, in field desorption, m/e 525 ($M^+$). The I.R. spectrum (KBr) shows absorptions at 1710 ($COCH_3$), 1670, 1620, and 1590 $cm^{-1}$ (quinone bands). The UV-Visible spectrum (in $CH_3OH$) shows absorption maxima at 227, 260, 284 (sh), 417, and 434 (sh) nm ($E^{1\%}_{1\,cm}$ 500, 330, 173, 167 and 146).

## Example 2

4'-O-methyl-11-deoxy-daunorubicin (III) (FCE/20291).

A solution of compound II (0.180 g; 0.34 mmoles) in anhydrous methanol (2.5 ml), anhydorus dioxan (6.9 ml), and ethyl orthoformate (0.18 ml) was treated with 1.3 M solution of bromine in chloroform (0.7 ml). After 2 hours, at 10°C, the resulting solution was poured in petroleum ether (18 ml) and diethyl ether (36 ml). The precipitate was filtered off, washed with diethyl ether and dried under vacuum.

The product thus obtained was dissolved in acetone (5.8 ml) and 0.25 M aqueous hydrogen bromide (5.8 ml) was added. The reaction mixture was kept overnight at room temperature.

To the reaction mxiture was added 20% aqueous sodium formate (1.35 ml). The solution was kept at 30°C for 24 hours. The resulting mixture was extracted with chloroform to eliminate impurities, then adjusted to pH 7.5 with saturated aqueous sodium bicarbonate solution and extracted with chloroform. The organic phase is washed with water, dried over anhydrous sodium sulphate, taken up in *n*-propanol and brought to a small volume. Addition of 2.1 N methanolic hydrogen chloride and of sufficient diethyl ether to cause precipitation afforded 0.135 g (0,23 mmoles) of 4'-O-methyl-11-deoxydoxorubicin (III) as its hydrochloride: m.p. 189—193°C, mass spectrum, in field desorption, m/e 541 ($M^+$). The I. R. spectrum (KBr) shows absorptions at 1725 ($COCH_2OH$), 1670, 1630, and 1590 $cm^{-1}$ (quinone bands). The UV-Visible spectrum (in $CH_3OH$) shows absorption maxima at 227, 259, 285 (sh), 418 and 436 (sh) ($E^{1\%}_{1\,cm}$ 673, 424, 221, 177 and 151).

## Example 3

4',6-Di-O-methyl-11-deoxy daunorubicin (IV) (FCE/20302).

Alkaline hydrolysis of 4',6-di-O-methyl-11-deoxy-N-trifluoroacetyl-daunorubicin (XIII) (0.180 g; 0.28 mmoles), following the procedure described in Example 1, yielded 4',6-di-O-methyl-11-deoxy-daunorubicin as its hydrochloride (IV) (0.030 g; 0.05 mmoles): m.p. 182—185°C (with decomposition); mass spectrum, in field desorption, m/e 539 ($M^+$). The I. R. spectrum (KBr) shows adsorptions at 1710 ($COCH_3$), 1670 and 1590 $cm^{-1}$ (quinone bands). The UV-Visible spectrum (in $CH_3OH$) shows absorption maxima at 223, 260 and 382 nm ($E^{1\%}_{1\,cm}$ 353, 326 and 109).

## Example 4

Preparation of 11-O-methyl-daunorubicin (VI) (FCE/20146).

N-Trifluoroacetyl-daunorubicin (XI) (1.5 g; 2.40 mmoles) was methylated with methyl iodide (60 ml), in the presence of silver oxide (10 g) following the procedure described in Example 1. Purification of the crude reaction mixture on a silica gel column using chloroform as eluant, yielded 11-O-methyl-N-trifluoroacetyl daunorubicin (XIV) (0.842 g; 1.32 mmoles) as the major product along with 4',11-di-O-methyl-N-trifluoroacetyl daunorubicin (XV) (0.270 g; 0.415 mmoles).

Compound XIV has: m.p. 136—138°C; $[\alpha]_D = +140°$ (c = 0.05 in $CH_3OH$); m/e 637 ($M^+$). The p.m.r. spectrum ($CDCl_3$) shows absorptions at 1.32 (d, $CH_3$—C—5'), 2.39 (s, $CH_3CO$), 3.85 (s, C—11—$OCH_3$), 4.03 (s, C—4—$OCH_3$), 5.23 (broad signal, C—7—H), 5.45 (broad signal, C—1'—H) and 13.95δ (s, C—6—OH).

Compound XV has: m.p. 103—105°C; $[\alpha]_D = +80°$ (c = 0.05 in $CH_3OH$); m/e 651 ($M^+$). The p.m.r. spectrum ($CDCl_3$) shows absorptions at 1.34 (d, $CH_3$—C—5'), 2.39 (s, $CH_3CO$), 3.53 (s, C—4'—$OCH_3$), 3.84 (s, C—11—$OCH_3$), 4.03 (s, C—4—$OCH_3$), 5.23 (broad signal, C—7—H), 5.48 (broad signal, C—1'—H), and 13.93δ (C—6—OH).

To a solution of compound XIV (0.400 g; 0.628 mmoles) in acetone (20 ml) was added dropwise 0.12 M aqueous sodium hydroxide (40 ml). The mixture was stirred at 0°C for 3 hours. The work-up is carried out according to the procedure described in Example 1 and yields 0.250 g (0.44 mmoles) of 11-O-methyldaunorubicin as its hydrochloride (VI): m.p. 186—187°C (with decomposition); mass spectrum, in field desorption, m/e 541 ($M^+$). The I. R. spectrum (KBr) shows absorptions at 1710 ($COCH_3$), 1670, 1620 and 1590 $cm^{-1}$ (quinone bands). The UV-Visible spectrum (in $CH_3OH$) shows absorption maxima at 230, 252, 281 (sh), 428 and 442 (sh) nm ($E^{1\%}_{1\,cm}$ 593, 355, 145, 180 and 175).

Example 5

4'-11-Di-O-methyldaunorubicin (VIII) FCE/20145.

Alkaline hydrolysis of 4',11-di-O-methyl-N-trifluoroacetyl-daunorubicin (XV) (0.250 g; 0.384 mmoles), following the procedure described in Example 4, yielded 4',11-di-O-methyl daunorubicin as its hydrochloride (VIII) (0.140 g; 0.237 mmoles): m.p. 192—194°C (with decomposition); mass spectrum, in field desorption, m/e 555 ($M^+$). The I. R. spectrum (KBr) shows absorptions at 1710 ($COCH_3$), 1670, 1620 and 1590 $cm^{-1}$ (quinone bands). The UV-Visible spectrum (in $CH_3OH$) shows absorption maxima at 230, 252, 284 (sh), 428 and 442 (sh) nm ($E^{1\%}_{1\,cm}$ 598, 430, 159, 159 and 159).

**Claims for the contracting states: BE CH DE FR GB LI NL SE**

1. A compound of the general formula I:

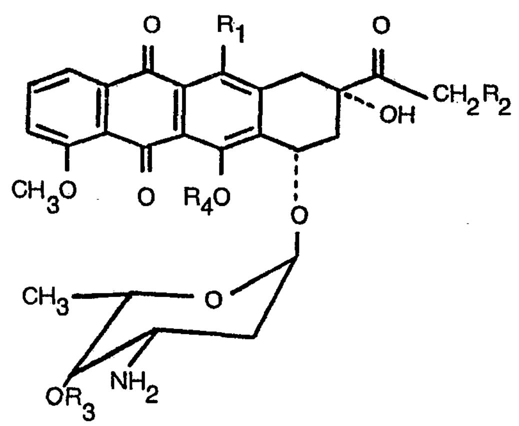

I

wherein $R_1$ represents a hydrogen atom or a methox group, $R_2$ represents a hydrogen atom or a hydroxy group, and each of $R_3$ and $R_4$ independently represents a hydrogen atom or a methyl group, with the proviso that $R_3$ and $R_4$ do not simultaneously represent hydrogen atoms and their pharmaceutically acceptable acid addition salts.

2. A compound according to claim 1, wherein $R_1$, $R_2$ and $R_4$ are hydrogen atoms and $R_3$ is a methyl group.

3. A compound according to claim 1, wherein $R_1$ and $R_4$ are hydrogen atoms and $R_2$ and $R_3$ are, respectively, a hydroxy group and a methyl group.

4. A compound according to claim 1, wherein $R_1$ and $R_2$ are hydrogen atoms and $R_3$, $R_4$ are methyl groups.

5. A compound according to claim 1, wherein $R_1$ is a methoxy group, $R_2$ and $R_4$ are hydrogen atoms and $R_3$ is a methyl group.

6. A compound according to claim 1, wherein $R_1$ is a hydrogen atom, $R_2$ is a hydroxy group and $R_3$, $R_4$ are methyl groups.

7. A compound according to claim 1, wherein $R_1$ is a methoxy group, $R_2$ a hydroxy group, $R_3$ a methyl group and $R_4$ is a hydrogen atom.

8. A process for the preparation of a compound according to any of the preceding claims, the process comprising N-trifluoroacetylating 11-deoxy-daunorubicin or daunorubicin, methylating the resultant N-trifluoroacetyl derivative respectively, removing the N-trifluoroacetyl group from the resultant methylated product, and optionally 14-brominating and subsequent hydrolysing to produce doxorubicin compounds wherein $R_2$=OH and, if desirable, reacting the respective products with acid to obtain the pharmaceutically acceptable acid addition salt.

9. A therapeutical composition containing at least one compound according to claim 1 to 7 as active ingredient along with usual pharmaceutically acceptable additives.

**Claims for the contracting state: AT**

1. A process for the preparation of a compound of general formula I

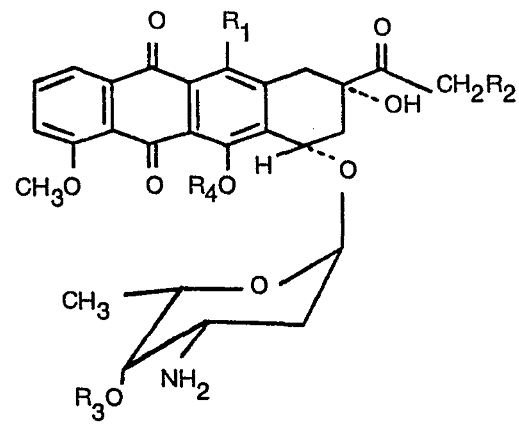

(I)

wherein $R_1$ represents a hydrogen atom or methoxy group, $R_2$ represents a hydrogen atom or a hydroxy group, and each of $R_3$ and $R_4$ independently represents a hydrogen atom or a methyl group, with the proviso that $R_3$ and $R_4$ do not simultaneously represent hydrogen atoms and their pharmaceutically acceptable acid addition salts, the process comprising N-trifluoroacetylating 11-deoxy-daunorubicin or daunorubicin, methylating the resultant N-trifluoroacetyl derivative respectively, removing the N-trifluoroacetyl group from the resultant methylated product, and optionally 14-brominating and subsequent hydrolysing to produce doxorubicin compounds wherein $R_2$=OH and, if desirable, reacting the respective products with acid to obtain the pharmaceutically acceptable acid addition salt.

2. A process according to claim 1, which comprises reacting the known 11-deoxy-daunorubicin or daunorubicin with trifluoroacetic anhydride in anhydrous chloroform to obtain their respective N-trifluoroacetyl derivatives which are successively and separately treated with methyl iodide in the presence of silver oxide, separating by chromatography on a silicagel column and using a chloroform-methanol gradient solution the so formed 4'-O-methyl-11-deoxy-N-trifluoroacetyl-daunorubicin from the contemporaneusly formed 4',6-di-O-methyl-11-deoxy-N-trifluoroacetyl-daunorubicin or the 11-O-methyl-daunorubicin from the 4'-11-di-O-methyl-daunorubicin, removing separately from each compound the protecting N-trifluoroacetyl groups by mild alkaline hydrolysis to obtain the free glycosidic bases ($R_2$ = H) which, by treatment with methanolic hydrogen chloride are isolated as their hydrochlorides and optionally reacted with bromine in chloroform to obtain their respective 14-bromo-derivatives, which, by subsequent hydrolysis at room temperature with a solution of sodium formate give rise to the doxorubicin derivatives of the general formula I ($R_2$ = OH) also isolated as their hydrochlorides.

3. A therapeutical composition containing at least one compound obtainable by the process according to claim 1 or 2 as active ingredient along with usual pharmaceutically acceptable additives.

**Revendications pour les Etats contractants: BE CH LI DE FR GB NL SE**

1. Composés de formule générale I:

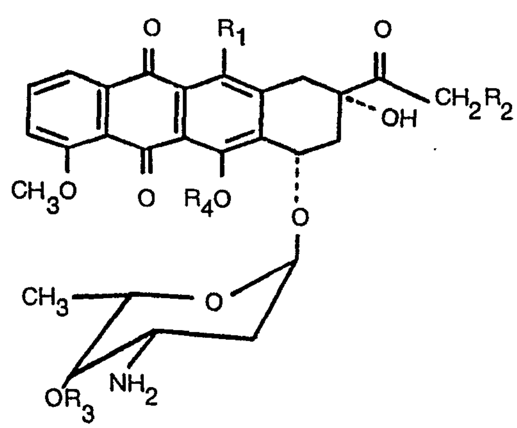

I

dans laquelle $R_1$ représente un atome d'hydrogène ou un groupe méthoxy, $R_2$ représente un atome d'hydrogène ou un groupe hydroxy, chacun de $R_3$ et de $R_4$ représente indépendamment un atome

d'hydrogène ou un groupe méthyle avec la condition que $R_3$ et $R_4$ ne représentent pas simultanément des atomes d'hydrogène et leurs sels d'addition acide pharmaceutiquement acceptables.

2. Composé selon la revendication 1 dans lequel $R_1$, $R_2$ et $R_4$ sont des atomes d'hydrogène et $R_3$ est un groupe méthyle.

3. Composé selon la revendication 1 dans lequel $R_1$ et $R_4$ sont des atomes d'hydrogène et $R_2$ et $R_3$ sont respectivement un groupe hydroxy et un groupe methyle.

4. Composé selon la revendication 1 dans lequel $R_1$ et $R_2$ sont des atomes d'hydrogène et $R_3$, $R_4$ sont des groupes méthyle.

5. Composé selon la revendication 1 dans lequel $R_1$ est un groupe méthoxy, $R_2$ et $R_4$ sont des atomes d'hydrogène et $R_3$ est un groupe méthyle.

6. Composé selon la revendication 1 dans lequel $R_1$ est un atome d'hydrogène, $R_2$ est un groupe hydroxy et $R_3$, $R_4$ sont des groupes méthyle.

7. Composé selon la revendication 1 dans lequel $R_1$ est un groupe méthoxy, $R_2$ est un groupe hydroxy, $R_3$ est un groupe méthyle et $R_4$ est un atome d'hydrogène.

8. Procédé pour la préparation d'un composé selon l'une quelconque des revendications 1 à 7 caractérisé en ce qu'on soumet à une opération de N-trifluoroacétylation la 11-désoxy-daunorubicine ou la daunorubicine, à une méthylation le dérivé N-trifluoroacétyle en résultant respectivement, on élimine le groupe N-trifluoroacétyle du produit méthylé résultant, et éventuellement on le soumet à une bromation en 14 et à une hydrolyse subséquente pour obtenir des composés de doxorubicine dans lesquels $R_2$=OH et, si on le souhaite, on fait réagir les produits respectifs avec de l'acide pour obtenir les sels d'addition acide pharmaceutiquement acceptables.

9. Composition thérapeutique caractérisée en ce qu'elle comprend au moins l'un des composés selon les revendications 1 à 7 comme ingrédient actif avec des additifs habituels pharmaceutiquement acceptables.

**Revendications pour l'Etat contractant: AT**

1. Procédé pour la préparation d'un composé de formule générale I:

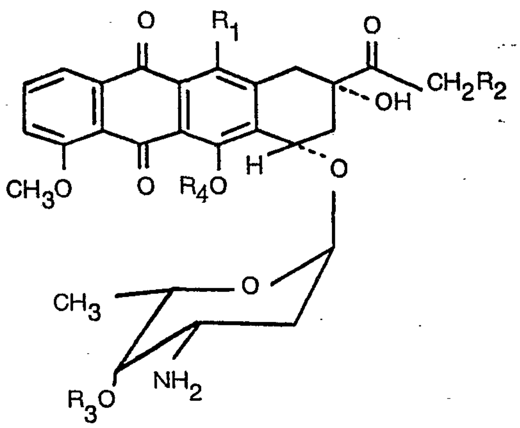

(I)

dans laquelle $R_1$ représente un atome d'hydrogène ou un groupe méthoxy, $R_2$ représente un atome d'hydrogène ou un groupe hydroxy, chacun de $R_3$ et de $R_4$ représente indépendamment un atome d'hydrogène ou un groupe méthyle avec la condition que $R_3$ et $R_4$ ne représentent pas simultanément des atomes d'hydrogène, et leurs sels d'addition acide pharmaceutiquement acceptables, ce procédé étant caractérisé en ce qu'on soumet à une opération de N-trifluoroacétylation la 11-désoxy-daunorubicine ou la daunorubicine, à une méthylation le dérivé N-trifluoroacétylé en résultant respectivement, on élimine le groupe N-trifluoroacétyle du produit méthylé résultant, et éventuellement on le soumet à une bromation en 14 et à une hydrolyse subséquente pour obtenir des composés de doxorubicine dans lesquels $R_2$=OH et, si on le désire, on fait réagir les produits respectifs avec un acide pour obtenir les sels d'addition acide pharmaceutiquement acceptables.

2. Procédé selon la revendication 1 caractérisé en ce qu'on fait réagir les corps connus 11-désoxy-daunorubicine ou daunorubicine avec de l'anhydride trifluoroacétique dans du chloroforme anhydre pour obtenir leurs dérivés respectifs N-trifluoroacétylés que l'on traite successivement et séparément avec de l'iodure de méthyle en présence d'oxyde d'argent, on sépare par chromatographie sur une colonne de gel de silice en utilisant une solution graduelle chloroforme-méthanol la 4'-O-méthyl-11-désoxy-N-trifluoroacétyl-daunorubicine ainsi formée de la 4',6-di-O-méthyl-11-désoxy-N-trifluoroacétyl-daunorubicine formée simultanément ou la 11-O-méthyl-daunorubicine de la 4'-11-di-O-méthyl-daunorubicine, on élimine séparément de chaque composé les groupes protecteurs N-trifluoroacétyles par une hydrolyse alcaline douce pour obtenir les bases libres glycosidiques ($R_2$ = H) que l'on isole sous forme de chlorhydrates par traitement avec de l'acide chlorhydrique méthanolique et

que l'on fait réagir, éventuellement, avec du brome dans du chloroforme pour obtenir les dérivés 14-bromo qui, par une hydrolyse subséquente à la température de la pièce, avec une solution de formiate de sodium, donnent les dérivés de doxorubicine de la formule générale I ($R_2 = OH$) que l'on isole aussi sous forme de chlorhydrates.

3. Composition thérapeutique contenant au moins un composé obtenu par le procédé selon l'une quelconque des revendications 1, 2, comme ingrédient actif avec des additifs habituels pharmaceutiquement acceptables.

**Patentansprüche für die Vertragsstaaten BE CH LI DE FR GB NL SE**

1. Verbindung der allgemeinen Formel (I)

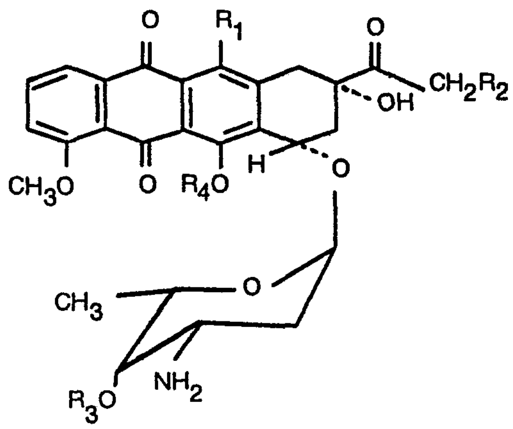

(I)

worin $R_1$ ein Wasserstoffatom oder eine Methoxygruppe, $R_2$ ein Wasserstoffatom oder eine Hydroxygruppe und jedes $R_3$ und $R_4$ unabhängig voneinander ein Wasserstoffatom oder ein Methylgruppe bedeuten, mit dem Proviso, dass $R_3$ und $R_4$ nicht gleichzeitig Wasserstoffatome bedeuten, und deren pharmaceutisch annehmbaren Säureadditionssalze.

2. Verbindung gemäss Anspruch 1, worin $R_1$, $R_2$ und $R_4$ Wasserstoffatome und $R_3$ eine Methylgruppe bedeuten.

3. Verbindung gemäss Anspruch 1, worin $R_1$ und $R_4$ Wasserstoffatome und $R_2$ und $R_3$ jeweils eine Hydroxygruppe und eine Methylgruppe bedeuten.

4. Verbindung gemäss Anspruch 1, worin $R_1$ und $R_2$ Wasserstoffatome und $R_3$ und $R_4$ eine Methylgruppe bedeuten.

5. Verbindung gemäss Anspruch 1, worin $R_1$ eine Methoxygruppe, $R_2$ und $R_4$ eine Wasserstoffatom und $R_3$ eine Methylgruppe bedeuten.

6. Verbindung gemäss Anspruch 1, worin $R_1$ ein Wasserstoffatom, $R_2$ eine Hydroxygruppe und $R_3$, $R_4$ Methylgruppen bedeuten.

7. Verbindung gemäss Anspruch 1, worin $R_1$ eine Methoxygruppe, $R_2$ eine Hydroxygruppe, $R_3$ eine Methylgruppe und $R_4$ ein Wasserstoffatome bedeuten.

8. Verfahren zur Herstellung einer Verbindung gemäss den vorhergehenden Ansprüchen, dadurch gekennzeichnet, dass man 11-Deoxy-daunorubicin oder Daunorubicin N-trifluoracetyliert, das jeweils gebildete N-Trifluoracetylderivat methyliert, die N-Trifluoracetylgruppe von dem gebildeten methylierten Produkt entfernt und gewünschtenfalls eine Bromierung in 14-Stellung vornimmt und anschliessend unter Bildung von Doxorubicinverbindungen, worin $R_2$=OH ist, hydrolysiert, und gewünschtenfalls das jeweilige Produkt mit einer Säure umsetzt, unter Ausbildung eines pharmazeutisch annehmbaren Säureadditionssalzes.

9. Arzneimittel, enthaltend wenigstens eine Verbindung gemäss Ansprüchen 1 bis 7 als aktiven Bestandteil, zusammen mit üblichen pharmazeutisch annehmbaren Additiven.

**Patentansprüche für den Vertragsstaat: AT**

1. Verfahren zur Herstellung einer Verbindung der allgemeinen Formel (I)

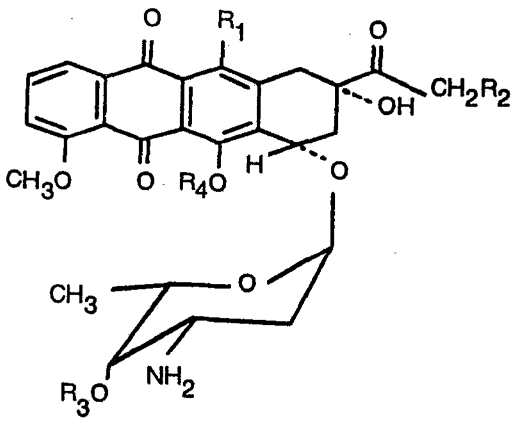

(I)

worin $R_1$ ein Wasserstoffatom oder eine Methoxygruppe, $R_2$ ein Wasserstoffatom oder eine Hydroxygruppe und jedes $R_3$ und $R_4$ unabhängig voneinander ein Wasserstoffatom oder eine Methylgruppe bedeuten, mit dem Proviso, dass $R_3$ und $R_4$ nicht gleichzeitig Wasserstoffatome bedeuten, und deren pharmazeutisch annehmbaren Säureadditionssalze, dadurch gekennzeichnet, dass man 11-Deoxydaunorubicin oder Daunorubicin N-trifluoracetyliert, das jeweils gebildete N-Trifluoracetylderivat methyliert, die N-Trifluoracetylgruppe von dem gebilderen methylierten Produkt entfernt und gewünschtenfalls eine Bromierung in 14-Stellung vornimmt und anschliessend unter Bildung von Doxorubicin-verbindungen, worin $R_2$=OH ist, hydrolysiert und gewünschtenfalls das jeweilige Produkt mit einer Säure umsetzt, unter Ausbildung eines pharmazeutisch annehmbaren Säureadditionssalzes.

2. Verfahren gemäss Anspruch 1, dadurch gekennzeichnet, dass man das bekannte 11-Deoxydaunorubicin oder Daunorubicin mit Trifluoressigsäureanhydrid in wasserfreiem Chloroform unsetzt, unter Erhalt des jeweiligen N-Trifluoracetylderivates, welches dann anschliessend und getrennt mit Methyljodid in Gegenwart von Silberoxid behandelt wird, worauf man dann chromatografisch über einer Kieselgelsäule unter Verwendung einer Chloroform-Methanol-Gradienten-Lösung das so gebildete 4'-O-Methyl-11-deoxy-N-trifluoracetyl-daunorubicin von dem gleichzeitig gebildeten 4',6-Di-O-methyl-11-deoxy-N-trifluoracetyl-daunorubicin oder das 11-O-Methyl-daunorubicin von dem 4',11-Di-O-methyl-daunorubicin trennt, getrennt von jeder Verbindung die schützende N-Trifluoracetylgruppe durch milde alkalische Hydrolyse unter Erhalt der freien glykosidischen Basen ($R_2$=H) entfernt, die dann durch Behandeln mit methanolischer Salzsäure als Hydrochloride isoliert werden und gewünschtenfalls mit Brom in Chloroform unter Erhalt der jeweiligen 14-Brom-derivate umgesetzt werden, welche dann durch anschliessende Hydrolyse bei Raumtemperatur mit einer Lösung von Natriumformiat Doxorubicinderivate der allgemeinen Formel (I) ($R_2$=OH), isoliert als Hydrochloride, ergeben.

3. Arzneimittel, enthaltend wenigstens eine Verbindung, erhältlich nach dem Verfahren gemäss Ansprüchen 1 oder 2, als aktiven Bestandteil, zusammen mit üblichen pharmazeutisch annehmbaren Additiven.